(19) Europäisches Patentamt
European Patent Office
Office européen des brevets

(11) Veröffentlichungsnummer : **0 051 205**
**B1**

(12) # EUROPÄISCHE PATENTSCHRIFT

(45) Veröffentlichungstag der Patentschrift : **02.01.86**

(51) Int. Cl.[4] : **C 07 K 7/08**, A 61 K 37/02, A 61 K 49/02, G 01 N 33/53

(21) Anmeldenummer : **81108536.4**

(22) Anmeldetag : **20.10.81**

(54) **Neues Tridekapeptid, Verfahren zu seiner Herstellung und Verwendung.**

(30) Priorität : **30.10.80 DE 3040825**

(43) Veröffentlichungstag der Anmeldung : **12.05.82 Patentblatt 82/19**

(45) Bekanntmachung des Hinweises auf die Patenterteilung : **02.01.86 Patentblatt 86/01**

(84) Benannte Vertragsstaaten : **AT BE CH DE FR GB IT LI LU NL SE**

(56) Entgegenhaltungen :
**Chemical Abstracts Band 94, Nr. 23 8. Juni 1981 columbus, Ohio, USA JUNG et al. "Synthesis, carbon-13 NMR and CD of the N-terminal tridecapeptide of the sequence of human fibroblast interferon" Seite 471, Spalte 2, Abstract Nr. 190074p**
**Chemical Abstracts Band 94, Nr. 9 2. März 1981 Columbus, Ohio, USA KNIGHT JR. et al. "Purification, amino acid composition and N-terminal amino acid sequence of human fibroblast interferon" Abstract Nr. 63504d**
**Chemical Abstracts Band 92, Nr. 11 17. März 1980 Columbus, Ohio, USA KNIGHT JR. et al. "Human fibroblast interferon: amino acid analysis and amino terminal amino acid sequence" Seite 439, Spalte 2, Abstract Nr. 92519b**
**Biochem. Biophys. Res. Commun. 103/4, 1981, 1149-1156**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Jung, Günther, Dipl.-Chem. Prof. Dr.**
**Ob der Grafenhalde 5**
**D-7400 Tübingen 1 (DE)**
Erfinder : **Brückner, Hans, Dipl.-Chem. Dr.**
**Lindenstrasse 55**
**D-7302 Ostfieldern/Nellingen (DE)**
Erfinder : **Swetly, Peter, Dr.**
**Hietzinger Hauptstrasse 40 B**
**A-1130 Wien (AT)**
Erfinder : **Bozler, Gerhard, Dipl.-Chem. Dr.**
**Alpenstrasse 38**
**D-7950 Biberach 1 (DE)**

**EP 0 051 205 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Tridekapeptid, nämlich H-Met-Ser-Tyr-Asn-Leu-Leu-Gly-Phe-Leu-Gln-Arg-Ser-Ser-OH, ein Verfahren zu seiner Herstellung nach der Festphasen-Synthese und seine Verwendung, beispielsweise als Hapten zur Kupplung mit einem Immunogen.

Hochgereinigtes Interferon, das weltweit als hochinteressante Substanz bearbeitet wird, steht bis heute nur in äußerst geringen Mengen für medizinische Untersuchungen zur Verfügung. Es war daher bisher nicht möglich, an Interferonproteinen strukturelle oder biochemische Untersuchungen durchzuführen. Nachdem nunmehr die N-terminale Sequenz des menschlichen Fibroblasteninterferons aufgeklärt werden konnte (Science *207*, 525-526 (1980)), wurde das obige Tridekapeptid erstmalig synthetisiert und seine Eingenschaften untersucht. Diese Aminosäuresequenz läßt sich ebenfalls aus der Deoxyribonukleinsäuresequenz des 5'-terminalen Endes des Interferon-Strukturgens oder der Ribonukleotidsequenz des 5'-Endes der Translationseinheit der Interferon-messenger Ribonukleinsäure, beginnend mit dem Initiationstriplet A.U.G. (A = Adenosin, U = Uridin, G = Guanosin) nach der Regel des genetischen Codes ableiten.

Im Nachfolgenden werden folgende in der Peptidchemie übliche Abkürzungen benützt :

H-Ser-OH = L-Serin
H-Arg-OH = L-Arginin
H-Gln-OH = L-Glutamin
H-Leu-OH = L-Leucin
H-Phe-OH = L-Phenylalanin
H-Gly-OH = Glycin
H-Asn-OH = L-Asparagin
H-Tyr-OH = L-Tyrosin
H-Met-OH = L-Methionin
Bzl = Benzylrest,
2,6-$Cl_2$-Bzl = 2,6-Dichlorbenzylrest,
Boc = tert. Butyloxycarbonylrest,
Tos = Tosylrest,
DMF = Dimethylformamid
DCC = N,N'-Dicyclohexylcarbodiimid
HOBt = 1-Hydroxybenzotriazol

Die Herstellung des neuen Tridekapeptids erfolgt nach der Festphasen-Synthese (siehe R. B. Merrifield in J. Amer. chem. Soc. *85*, 2149-2154 (1963)). Bei dieser wird die C-terminale Aminosäure eines Peptids über ihre Carboxylgruppe an ein Polymer als unlöslicher Träger angelknüpft. Dies geschieht entweder vorzugsweise über ein Alkali- oder Erdalkalisalz der entsprechenden N-geschützen Aminosäure, wenn ein Chlormethyl-Harz verwendet wird, oder durch Aktivierung des Carboxylfunktion der entsprechenden N-geschützten Aminosäure, beispielsweise mit N,N'-Dicyclohexylcarbodiimid, wenn ein Hydroxymethyl-Harz verwendet wird. Anschließend erfolgt des schrittweise Aufbau des Peptids durch stufenweisen Anbau der einzelnen Aminosäuren an das N-Ende des Peptidfragments (Abb. 1) und anschließende Abspaltung des verwendeten Aminoschutzrestes. Die Anknüpfung der nächstfolgenden N-geschützten Aminosäure an die nun freie und reaktionsfähige endständige Aminogruppe des Peptidfragments erfolgt durch Aktivierung ihrer Carboxylfunktion oder über einen reaktiven Ester. Nach erfolgtem Aufbau der Sequenz wird das Peptid vom Harz abgespalten.

Die Anwesenheit der Aminosäuren Serin, Tyrosin und Arginin erfordert hierbei zusätzlichen Schutz der Seitenfunktionen, z. B. durch ihre Überführung in die entsprechenden O-Benzylserin-, O-2,6-Dichlorbenzyl-tyrosin- und $N^g$-Tosyl-arginin-Derivate.

Bei der erfindungsgemäßen Festphasen-Synthese wird vorzugsweise ein quellbares Polymer, z. B. chlormethyliertes Polystyrol, das vorzugsweise mit 1 % Divinylbenzol quervenetzt ist, verwendet (PS-1 % DVB).

Besonders vorteilhaft wird erfindungsgemäß das Verfahren in der Weise durchgeführt, daß Serin als erste Aminosäure in seiner geschützten Form, wie z. B.

Boc—Ser (Bzl)—OH

mit Hilfe seines Cäsium-Salzes, das man zweckmäßigerweise durch Umsetzung der oben erwähnten geschützen Aminosäure mit Cäsiumcarbonat oder Cäsiumhydroxid erhält, an chlormethyliertes Polystyrol in einem Lösungsmittel, vorzugsweise in einem aprotischen Lösungsmittel wie Dimethylformamid, verestert wird. Anschließend wird die verwendete Aminoschutzgruppe abgespalten, z. B. die N-tert.Butoxycarbonylgruppe mit einer Säure, z. B. mit 50 %iger Trifluoressigsäure in Dichlormethan, vorzugsweise nach Vorwaschen mit Dichlormethan.

Pro anschließendem Synthese-Zyklus wird mit einem Überschuß der entsprechenden Boc-Aminosäure bzw. deren aktiviertem Ester, z. B. mit der 3- bis 6-fachen Menge, gegebenenfalls in Gegenwart eines Kupplungsreagenzes wie N,N'-Dicyclohexyl-carbodiimid gekuppelt, wobei dieser Vorgang mehrmals, z.

B. 2 bis 5 Mal, ohne vorherige Prüfung auf noch gegebenenfalls vorhandene freie Aminogruppen mit einem weiteren vergleichbaren Überschuß der entsprechenden Boc-Aminosäure und Kupplungsreagenz oder deren aktiviertem Ester wiederholt wird (Nachkupplung). Nach Vorwaschen wird der Boc-Schutzrest wie vorstehend beschrieben abgespalten.

Dieser Synthese-Zyklus wird gemäß Abb. 1 solange mit den entsprechenden N-geschützten Aminosäuren, bzw. deren reaktiven Estern wiederholt, bis das gewünschte geschützte Tridekapeptid-Harz erhalten ist.

Die Aminosäuren-Derivate Boc-Gln und Boc-Asn werden hierbei vorzugsweise mit Hilge eines ihrer reaktiven Ester, z. B. über ihren p-Nitrophenylester, gekuppelt. Desweiteren kann bei den Kupplungen und den Nachkupplungen 1-Hydroxy-benzotriazol als Katalysator zugesetzt werden.

Nach jedem Synthese-Zyklus wird mit Essigsäureanhydrid/N-Methylmorpholin acetyliert, um eine gegebenenfalls bei der Kupplung nicht umgesetzte Aminogruppe des Peptidfragments für die nächste Kupplung zu blockieren. Außerdem wurde nach etwa dem neunten Synthese-Zyklus ein stärkeres Quellverhalten des Peptidharzes beobachtet, sodaß insbesondere bei der Behandlung mit Trifluoressigsäure/Dichlormethan wesentlich mehr Reagenzlösung erforderlich ist als in den ersten Synthesezyklen.

Nach durchgeführter Synthese werden durch Einleiten von Bromwasserstoff in die Suspension des Peptidharzes in Trifluoressigsäure unter Zusatz von Resorcin und Thioanisol Boc- und Bzl-Reste entfernt und das gebildete $N^g$-Tosyl-tridekapeptid von der verwendeten Festphase abgespalten. Das so erhaltene, durch Umfällen gereinigte $N^g$-Tosyl-tridekapeptid wird zur Abspaltung der Tosylgruppe mit Natrium in flüssigem Ammoniak behandelt. Zur weiteren Reinigung wird das so erhaltene Tridekapeptid umgefällt, z. B. aus Eisessig/Ether und Dimethylformamid/Aceton, über eine Gelsäule, z. B. Sephadex® G 25, mit Eisessig/Wasser (1/1) chromatographiert und abschließend einer multiplikativen Gegenstromverteilung, z. B. mit 1-Butanol/5 % Essigsäure/1-Propanol (10/10/2) als Zweiphasen-System, unterworfen.

Das nachstehende Beispiel soll die Erfindung näher erläutern :

I. Herstellung des geschützten Tridekapeptids am Träger :

Stufe 1

6,0 g (20 mmol) Boc-Ser(Bzl)-OH werden in 70 ml Ethanol/Wasser (7 : 2) am pH-Meter mit einer wässrigen Lösung von ca. 3.2 g (10 mmol) Cäsiumcarbonat bis pH 6-7 neutralisiert. Nach Abdampfen im Vakuum wird das restliche Wasser azeotrop mit Benzol entfernt. Nach Trocknen bei 0.1 mbar über Diphosphorpentoxid/Kaliumhydroxid wird das glasig erstarrte Cäsiumsalz in 50 ml Dimethylformamid gelöst und mit 15 g Merrifield-Harz (Polystyrol-1 % Divinylbenzol, 0,9 meq. Cl/g) bei 50 °C 48 Stunden langsam gerührt. Dann wird das Harz gewaschen mit je 3 × 50 – 100 ml Dimethylformamid, Dimethylformamid/Wasser (9 : 1), Ethanol 95 %, Ethanol wasserfrei, Dioxan, Ethanol, Chloroform Ethanol, Dichlormethan, Methanol und im Vakuum über Phosphorpentoxid getrocknet. Die Beladung beträgt aufgrund der Aminosäureanalyse 0,25 mmol/g.

Stufe 2

Das mit Boc-Ser(Bzl)-OH veresterte Merrifield-Harz wird mit 2 × 100 ml Dichlormethan gewaschen. Zur Abspaltung der Boc-Schutzgruppe werden 100 ml Dichlormethan/Trifluoressigsäure Gemisch (v/v, 1 : 1) zugegeben ; nach 5 Minuten wird abgesaugt und mit frischer Reaktionslösung derselben Zusammensetzung versetzt. Nach 20 Minuten wird je dreimal mit je 100 ml Dichlormethan und dann Dichlormethan/Dioxan (v/v, 2 : 1) gewaschen. Anschließend wird mit 100 ml Triethylamin/Chloroform (v/vn 1 : 9) die Aminofunktion des Serinrestes deprotoniert. Nach 2 Minuten wird die Aminlösung durch frische Lösung ersetzt. Nach 5 Minuten wird je 100 ml Chloroform einmal und mit Dichlormethan dreimal gewaschen. Zur Kupplung wird das Harz mit 6 g (20 mmol) Boc-Ser(Bzl)-OH in 50 ml Dichlormethan 10 Minuten lang suspendiert. Dann werden 20 mmol (4,12 g) DCC in 20 ml Dichlormethan zugegeben. Zur Nachkupplung werden nach 30 Minuten weitere 10 mmol Boc-Ser(Bzl)-OH und 10 mmol DCC, jeweils in etwas Dichlormethan gelöst, zugefügt. Nach einer Kupplungszeit von insgesamt 2 Stunden wird je dreimal mit je 100 ml Dichlormethan und DMF gewaschen. Dann werden 5 ml Essigsäureanhydrid in 40 ml DMF, zugefügt. Nach 20 Minuten wird je dreimal mit je 100 ml DMF, Ethanol und Dichlormethan gewaschen.

Stufe 3

Kupplung mit 8,6 g (20 mmol) Boc-Arg(Tos)-OH, Nachkupplung mit 4,3 g (10 mmol) Boc-Arg(Tos)-OH analog Stufe 2. Das in Dichlormethan schwerlösliche Boc-Arg(Tos)-OH wird zuerst in etwas DMF gelöst und dann erst zu Dichlormethan gegeben.

Stufe 4

Nach Abspaltung der BOC-Schutzgruppe analog Stufe 2 werden 7,35 g (20 mmol) Boc-Gln-ONp in 70

ml DMF gelöst und zum Tripeptidharz gegeben. Nach 1/2-stündiger Reaktionsdauer werden 20 mmol HOBt zugefügt und zur Nachkupplung nach weiteren 60 Minuten 14,7 g (40 mmol) Boc-Gln-OH, 40 mmol HOBt und 20 mmol N,N'-Dicyclohexyl-carbodiimid. Nach 30 Minuten Reaktionsdauer wird das Harz 3 mal mit DMF gewaschen, dann 10 Minuten mit einer Mischung von 3 ml Essigsäureanhydrid in 40 ml DMF und 1,5 g N-Methylmorpholin in 40 ml DMF reagieren lassen, dann je 3 mal mit DMF, 3 mal mit abs. Ethanol und 3 mal mit Dichlormethan gewaschen.

Stufe 5

Nach Abspaltung der Boc-Schutzgruppe und Neutralisation analog Stufe 2 werden 7,5 g (30 mmol) Boc-Leu-OH × $H_2O$ (vorgetrocknet) in 20 ml Dimethylformamid gelöst, zum Tetrapeptid-Harz gegeben und 30 ml (30 mmol) einer 1 molaren N,N'-Dicyclohexyl-carbodiimid-Lösung in Dichlormethan zugegeben. Nach 1,5-stündiger Reaktionsdauer werden zur Nachkupplung 7,5 g (30 mmol) Boc-Leu-OH × $H_2O$, 30 mmol einer 1 molaren N,N'-Dicyclohexyl-carbodiimid-Lösung in Dichlormethan und 4,05 g (30 mmol) HOBt zugefügt. Nach 2 1/4 stündiger Reaktionsdauer wird 1 mal mit Dichlormethan, 2 mal mit Dioxan und 3 mal mit Dimethylformamid gewaschen. Anschließend erfolgt die Behandlung mit 6 ml Essigsäureanhydrid in 40 ml Dimethylformamid und 3 g N-Methylmorpholin in 40 ml Dimethylformamid (Reaktionszeit : 10 Minuten). Dann wird je 3 mal mit Dimethylformamid, 3 mal mit Ethanol und 3 mal mit Dichlormethan gewaschen.

Stufe 6

Kupplung mit 5,3 g (20 mmol) BOC-Phe-OH, Nachkupplung mit 5,3 g (20 mmol) BOC-Phe-OH analog Stufe 2.

Stufe 7

Kupplung mit 3,5 g (20 mmol) BOC-Gly-OH, Nachkupplung mit 3,5 g (20 mmol) BOC-Gly-OH analog Stufe 2.

Stufe 8

Kupplung mit 5,0 g (20 mmol BOC-Leu-OH × $H_2O$, Nachkupplung mit 4,25 g (17 mmol) BOC-Leu-OH × $H_2O$ analog Stufe 5.

Stufe 9

Kupplung mit 5,0 g (20 mmol) BOC-Leu-OH × $H_2O$, Nachkupplung mit 4,25 g (17 mmol) BOC-Leu-OH × $H_2O$ analog Stufe 5.

Stufe 10

Kupplung mit 7,0 g (20 mmol) BOC-Asn-ONp, Nachkupplung mit 7,0 g (20 mmol) BOC-Asn-Onp analog Stufe 4.

Stufe 11

Kupplung mit 8,8 g (20 mmol) BOC-Tyr(O-2,6-$Cl_2$-Bzl)-OH, Nachkupplung mit 8,8 g (20 mmol) BOC-Tyr(O-2,6-$Cl_2$-Bzl)-OH analog Stufe 2.

Stufe 12

Kupplung mit 6,0 g (20 mmol) BOC-Ser(Bzl)-OH, Nachkupplung mit 6,0 g (20 mmol) BOC-Ser(Bzl)-OH analog Stufe 2.

Stufe 13

Kupplung mit 5,0 g (20 mmol) BOC-Met-OH, Nachkupplung mit 5,0 g (20 mmol) BOC-Met-OH analog Stufe 2.

II. Abspaltung des Peptids vom Träger :

Das Peptid-Harz wird nach dem letzten Waschen mit Dichlormethan 15 Stunden über Diphosphorpentoxid/Kaliumhydroxid im Vakuum getrocknet. Anschließend wird das $Arg^{11}$(Tos)-Tridekapeptid mit HBr in Trifluoressigsäurelösung (250 ml) bei Raumtemperatur abgespalten. Die Waschflaschen für das HBr-Gas und das Reaktionsgefäß enthalten je 3 % Resorcin und Thioanisol. Nach 120 Minuten wird

vom Harz abgesaugt und die Trifluoressigsäure-Lösung sofort am Rotationsverdampfer eingeengt (20 °C Badtemperatur). Der ölige Rückstand wird in 150 ml Eisessig/Wasser (3 : 1) gelöst und bei 40 °c Badtemperatur wieder eingeengt. Der Rückstand wird in ca. 800 ml tert. Butanol/Wasser (1 : 1) gelöst und lyophilisiert. Es resultiert ein farbloses Lyophilisat. Das Lyophilisat ist in Wasser und den meisten organischen Lösungsmitteln schwerlöslich. Es wird aus Eisessig/Wasser und Eisessig/Ether umgefällt, und es erstarrt in vielen Lösungsmitteln gelartig. Eine weitere Umfällung wird aus Dimethylformamid/Methanol/Chloroform (7 : 4 : 3)-Lösung durch Zusatz von Ether vorgenommen. Die gallertartige Ausfällung läßt sich auf der Fritte absaugen und wird über Diphosphorpentoxid/Kaliumhydroxid im Vakuum getrocknet.

Ausbeute : 15,65 g $Arg^{11}$ (Tos)-Tridekapeptid.

III. Abspaltung der Tosyl-Schutzgruppe :

5 g trockenes $Arg^{11}$ (Tos)-Tridekapeptid werden in 500 ml flüssigem Ammoniak mit einer $Na/NH_3$-Lösung versetzt bis zur stabilen Blaufärbung. 15 Sekunden danach wird $NH_4Cl$ zugegeben. Dann wird der Ammoniak mit trockenem $N_2$ abgeblasen und der Rückstand in Eisessig/Wasser (1 : 1) gelöst. Nach Einengen am Rotationsverdampfer bis auf etwa 30 ml wird die Peptidlösung in Zentrifugenbechern in 120 ml Wasser eingerührt. Dabei fällt das Tridekapeptid als dicker, farbloser Niederschlag aus, der dreimal unter Zentrifugieren mit Wasser gewaschen wird.

IV. Chromatographische Reinigung des Tridekapeptids :

Das rohe Tridekapeptid wird an Sephadex® G 25 coarse chromatographiert. Säule : 5 × 90 cm ; Elutionsmittel : Eisessig/Wasser (1 : 1) ; Fließgeschwindigkeit 7.15 ml/10 min. Das noch feuchte Tridekapeptid wird durch Zugabe des gleichen Volumens an Eisessig gelöst. Die ca. 75 ml Lösung werden auf einmal aufgetragen. Die 18 ml-Fraktionen werden dünnschichtchromatographisch auf Kieselgel-Platten im System 1-Butanol/Eisessig/Wasser (3 : 1 : 1) untersucht (Sprühreagenz : Chlor/4,4'-Bis(Dimethylamino)diphenyl-methan). Das Peptid erscheint im Elutionsvolumen 422-792 ml. Die vereinigten Fraktionen werden im Vakuum eingeengt und der Rückstand in ca. 20 ml Eisessig gelöst. Diese Lösung wird langsam unter Rühren zu wasserfreiem Ether getropft. Das Peptid fällt dabei völlig farblos in rasch sedimentierenden Flocken aus. Es wird in der Zentrifuge dreimal mit wasserfreiem Ether gewaschen. Nach Trocknen im Vakuum bleiben 3,1 g pulvriges Produkt übrig, das zur weiteren Reinigung aus Dimethylformamid mit Aceton ausgefällt wird. Dünnschichtchromatographische Untersuchungen im obengenannten System zeigten ein weitgehend reines Peptid an. Die Aminosäureanalyse lieferte die erwarteten Werte und einen Peptidgehalt von 43 %.

V. Reinigung des Tridekapeptids durch multiplikative Gegenstromverteilung :

0,5 g Tridekapeptid werden unter Rühren im Verteilungssystem 1-Butanol/5 % Essigsäyre/1-Propanol (5 : 5 : 1) gelöst. Die Lösung wird auf die ersten 15 Rohre einer Craig-Apparatur (Elemente à 10 ml) über 120 Stufen verteilt. Lipophilere Verunreinigungen, aufgrund der Aminosäureanalyse vermutlich N-Acetylpeptide, laufen an der Front, und salzhaltige Verunreinigungen bleiben am Start in der wäßrigen Phase. Nach dünnschichtchromatographischer Untersuchung (System : 1-Butanol/Eisessig/Wasser (3 : 1 : 1), $R_f$ 0.25) erscheint völlig reines Tridekapeptid mit $r_{max} = 45$.

Ausbeute : 150 mg.

Die Identität des gemäß vorstehenden Beispiels erhaltenen Tridekapeptids wurde durch Aminosäureanalyse, $^{13}C$-NMR-spektroskopische Untersuchungen sowie eine Racematbestimmung bewiesen.

Die Aminosäureanalyse wurde in einem Biotronik-Aminosäure-analysator LC 600 E durchgefüHrt. Die Hydrolyse erfolgte mit 6 N Salzsäure bei 110 °C und über 18 bis 72 Stunden. H-Ser-OH, H-Met-OH und H-Tyr-OH wurden bei der Hydrolyse teilweise zerstört, die Leu-Leu-Bindung wurde erst nach 72 Stunden vollständig gespalten. Ohne Korrektur der Hydrolyseverluste wurde die nachstehende Zusammensetzung an Aminosäuren festgestellt :

(Siehe Tabelle 1 Seite 6 f.)

Tabelle 1

| Aminosäure | Zahl der Aminosäurereste gefunden | berechnet |
|---|---|---|
| Asp | 1,00 | 1 |
| Ser | 2,71 | 3 |
| Glu | 1,06 | 1 |
| Gly | 1,07 | 1 |
| Met | 0,89 | 1 |
| Leu | 2,60 | 3 |
| Tyr | 0,77 | 1 |
| Phe | 1,00 | 1 |
| Arg | 1,00 | 1 |

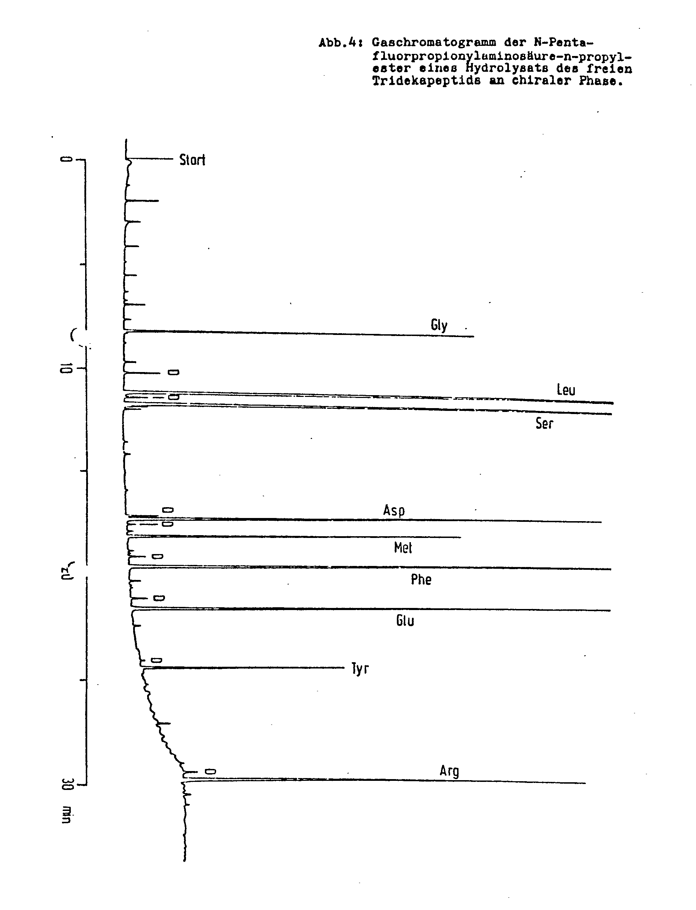

Die gaschromatographische Untersuchung der N-Pentafluorpropionylaminosäure-n-propylester des Totalhydrolysats des Tridekapeptids an der chiralen Phase Chirasil-Val (J. Chromatogr. *146*, 197 (1978)) erwies eine sehr hohe enantiomere Reinheit der Aminosäurereste des Tridekapeptids (siehe Abb. 4).

Die $^{13}C$-NMR-Spektren wurden im NMR-Spektrometer WH-90 der Firma Bruker-Physik, Karlsruhe, bei 30 °C vermessen. Wegen der Schwerlöslichkeit des Tridekapeptids wurde eine $^{12}C$, $^{2}H$-Dimethylsulfoxidlösung verwendet. Bei einer Konzentration von 60 mg/ml und bei 12-stündiger Akkumulationszeit wurde ein relativ gut aufgelöstes $^{13}C$-NMR-Spektrum (20.115 MHz) erhalten, das eindeutig die Identität des neuen Tridekapeptids mit der Sequenz 1 bis 13 beweist (Abbildung 2). Besonders gut zu erkennen sind die singulär auftretenden, charakteristischen Signale der S-Methylgruppe von Methionin (14,6 ppm), der 6 Methylgruppen (21.6-23.2 ppm) und der C$\gamma$-Kohlenstoffe (24.1 ppm) dert drei Leucinreste im Hochfeldteil des Spektrums (Abb. 3a). Im Aromatenbereich bereitet die Zuordnung der Aromatensignale von Phenylalanin und Tyrosin ebenfalls keine Schwierigkeiten (Abb. 3b). Ferner sind die Resonanzsignale bei 155.8 ppm (phenolischer C-4-Kohlenstoff des Tyrosins) und bei 157.5 ppm (Guanidino-Kohlenstoff des Arginins) strukturbeweisend. Dabei ist anzumerken, daß die Intensitäten dieser typischen Signale vergleichbar sind, obgleich diese Aminosäuren positionell in der Sequenz weit auseinanderliegen (siehe Abb. 1). Auch der Carbonylteil des Spektrums weist, wie erwartet, 15 Signale vergleichbarer Intensitäten auf (Abb, 3c). Dies zeigt das Vorliegen eines sehr einheitlichen Peptids.

Das Tridekapeptid wurde auch anhand seines CD-Spektrums in Trifluorethanol- und Trifluorethanol/Hexafluoraceton-Sesquihydrat-Lösungen charakterisiert. Das Peptid weist eine helikale Konformation auf, die negative Cotton-Effekte mit Maxima bei 223 und 207 nm erzeugt.

Das neue Tridekapeptid kann als Hapten eingesetzt werden, das mit einem natürlichen Protein, wie z. B. Humanserumalbumin, Rinderserumalbumin, Eialbumin oder mit einem synthetischen Polypeptid wie z. B. poly-L-Lysin, poly-L-Alanyl-L-lysin oder mit anderen Trägern wie z. B. modifizierte Dextrane nach bekannten Methoden gekoppelt wird. Das Peptid läßt sich auch über andere Polyamine, wie z. B. 1,6-Diaminohexan, verknüpfen. Ein so hergestellte Immunogen oder das Tridekapeptid selbst können mit bekannten Methoden zur Erzeugung von Antiseren bzw. Antikörpern gegen menschliches Fibroblasteninterferon eingesetzt werden.

Das neue Tridekapeptid kann an Stelle des menschlichen Fibroblasteninterferons in geeigneter Zubereitung zur Therapie verwendet werden.

Das neue Tridekapeptid ist als Tracer für die immunologische Bestimmung von menschlichem Fibroblasteninterferon geeignet. Dazu kann es einerseits direkt nach bekannten Methoden, wie z. B. mit radioaktivem Jod oder anderen geeigneten Markern, z. B. Enzymen wie Peroxydasen oder fluoreszierenden Verbindungen, markiert werden. Andererseits läßt es sich auch in Form der oben erwähnten Kupplungsprodukte mit den gleichen oder ähnlichen Methoden markieren und als Tracer verwenden.

Das neue Tridekapeptid eignet sich ferner in Form eines Kupplungsproduktes mit einem immobilen Träger, wie z. B. Dextranen, Sepharose oder Polystyrol, oder modifizierten anorganischen Trägern, wie z. B. Biogel oder CPG-10, zur Isolierung und Reinigung von Antikörpern gegen menschliche Fibroblasteninterferon. Derart gewonnene Antikörper gestatten in bekannter Weise die Reinigung und Isolierung von menschlichen Fibroblasteninterferon.

Das nach der schrittweisen Synthese am Träger befindliche voll geschützte Tridekapeptid kann vom Träger auch in geschützter Form abgespalten werden, z. B. durch Hydrazinolyse, Verseifung oder Umesterung. Diese abgespaltenen, partiell bzw. voll geschützten Tridekapeptidderivate stellen außerdem wertvolle Zwischenprodukte für die Synthese von höheren Peptiden des Fibroblasteninterferons oder des Fibroblasteninterferons selbst dar.

Die nachstehenden Beispiele beschreiben verschiedene Anwendungsmöglichkeiten des Tridekapeptids :

### Beispiel A

Kupplung des Tridekapeptids mit poly-L-Lysin zur Erzeugung eines Immunogens, das zur Gewinnung von Antiseren gegen menschliches Fibroblasteninterferon geeignet ist

46,9 mg poly-L-Lysin-hydrobromid (Molmasse 37.300 ; 0,223 mmol Aminofunktionen) werden in 0,3 ml Wasser von pH ca. 4 gelöst und unter Rühren mit 5 µl Triethylamin auf pH ca. 9,5 eingestellt. Dann gibt man 2 N Salzsäure bis pH 7.5 zu und fügt anschließend 57.3 mg (0,038 mmol) Triedekapeptid (Molmasse 1516), gelöst in 2,5 ml Dimethylformamid unter Erwärmen, und 389,3 mg (1,890 mmol) N,N'-Dicyclohexylcarbodiimid, gelöst in 0,5 ml Dimethylformamid, zu. Nach 24 Stunden bei Raumtemperatur wird das Konjugat mit Ether ausgefällt und mit Ether gewaschen. Anschliessend wird in Wasser unter Zusatz von etwas Ameinsensäure gelöst und 48 Stunden gegen Wasser dialysiert (Ausschlußgrenze 8 000) und dann lyophilisiert.

Ausbeute : 52 mg ; Beladung 17,8 Mol Tridekapeptid pro 1 Mol poly-L-Lysin.

### Beispiel 8

Jodmarkierung des Kupplungsproduktes des Tridekapeptids mit poly-L-Lysin

Von dem trockenen Kupplungsprodukt mit poly-L-Lysin werden 2 mg eingewogen und in 52 µl einer Mischung von 25 µl Wasser, 25 µl Dimethylsulfoxid, 2 µl Eisessig gelöst. Diese Lösung wird mit einem Natrium-Borat-Puffer verdünnt. 12 µl davon mit einer Konzentration von Borat von 0,1 m pH 9,0 und vom Kupplungsprodukt mit 10 µg/µl werden bei einer Temperatur von 0 °C (auf Eis) in ein Reaktionsgefäß gefüllt, in welchem das BOLTON-HUNTER-Reagenz (N-Hydroxy-succinimidester der mit $^{125}$J-jodierten-p-Hydroxyphenylpropionsäure) aus Benzol getrocknet war.

Die Reaktion wird nach einer Stunde gestoppt durch Zugabe von 90 µl 0,3 M Glycin in 0,1 M Natrium-Borat, pH 9,0. Nach weiteren 10 Minuten wird das gesamte Volumen nun auf eine Chromatographiesäule gegeben (7 ml Bettvolumen, Sephadex® G 25 Medium (Pharmacia, Uppsala), welche mit 50 mM Natriumphosphat pH 7,5 und 0,25 % Gelatine äquilibriert worden war und in diesem Puffer auch entwickelt wird. Die hochmolekulare markierte Substanz wird hierbei von niedrigmolekularen radioaktiven Reaktionsprodukten getrennt und erscheint in den ersten Fraktionen des Voidvolumens.

Eine bessere Ausbeute — verglichen mit diesen ursprünglichen Vorschriften von BOLTON und HUNTER — wird erreicht, wenn nach dem Ende der Reaktion die Lösung auf einen sauren pH gebracht wird. Nach der Zugabe von Glycin wird der pH von 9 auf 4 eingestellt, durch eine weitere Zugabe von 100 µl 20 mM Natrium-Acetat pH 4,0. Die Chromatographiesäule ist entsprechend mit 20 mM Natrium-Acetat pH 4,0 und 0,25 % Gelatine vorbehandelt und wird in diesem Puffer entwickelt.

Ausgehend von einer Radioaktivität von 0,2 mCi eines BOLTON-HUNTER-Reagenzes mit der spezifischen Radioaktivität von 2 000 Ci/mmol wird eine Markierung von mindestens $10^4$ cpm (counts per minutem/µg Kupplungsprodukt erreicht.

### Beispiel C

Herstellung von Kupplungsprodukten des Tridekapeptids mit AH-Sepharose® 4B zur Isolierung und Reinigung von Antikörpern gegen menschliches Fibroblasteninterferon

Die freie Carboxygruppe des Tridekapeptids wird an die Aminogruppen von AH-Sepharose® 4B (Pharmacia) mit Hilfe von Carbodiimid gekuppelt. Dazu werden 500 mg AH-Sepharose 4B in 5 ml Dimethylformamid/Wasser pH 4,5 suspendiert und mit 30,3 mg (0,02 mmol) Tridekapeptid gelöst in DMF versetzt. Nach tropfenweiser Zugabe von 300 mg N-Ethyl-N'-(3-dimethylaminopropyl)-carbodiimid-hydrochlorid (EDC) in etwas Wasser wird die Reaktionsmischung 12 Stunden bei Raumtemperatur geschüttelt. Anschließend wird das Gel solange mit DMF/Wasser (4 : 1), Dioxan/Wasser (1 : 1) und dann mit Wasser gewaschen, bis das Filtrat frei ist von überschüssigem EDC und Tridekapeptid, sowie N-Ethyl-N'-dimethylaminopropyl-harnstoff.

### Beispiel D

Herstellung von Kupplungsprodukten des Tridekapeptids an Lysin-Sepharose® 4B zur Affinitätschromatographie

Kupplung von 30,3 mg (0,02 mmol) Tridekapeptid mit 500 mg Lysin-Sepharose® 4B (Pharmacia) analog Beispiel C.

Beispiel E

Herstellung von Kupplungsprodukten des Tridekapeptids an Aminopropyl-CPG-10 zur Affinitätschromatographie

An je 500 mg Controlled-Pore Glass CPG-10 belegt mit Aminopropylgruppen der Porengrößen 75 sowie 120 Ångström Durchmesser werden je 30 mg Tridekapeptid mit Hilfe von Carbodiimid gekuppelt. Reaktionsbedingungen analog Beispiel C.

Beispiel F

Kupplung des Tridekapeptids an 1,6-Diaminohexan

Zu 15,16 mg (10 µmol) Tridekapeptid und 0,58 mg (5 µmol) 1,6-Diaminohexan in 2 ml DMF werden 20,6 mg (100 µmol) N,N'-Dicyclohexylcarbodiimid gegeben. Der Fortschritt der Reaktion wird dünnschichtchromatographisch mit dem System 1-Butanol/Eisessig/Wasser (3 : 1 : 1) verfolgt. Nach beendeter Kupplung wird die Reaktionslösung unter Rühren zu 5 ml Ether zugetropft. Der ausgefallene Niederschlag wird unter Zentrifugieren 2 × mit Ether gewaschen. Anschließend wird das Produkt zur Abtrennung des Monoadduktes an Sephadex® LH 20 chromatographier (Elutionsmittel : DMF, Säule 1 × 50 cm, Detektion durch DC). Alternativ kann das Produkt durch präparative Dünnschichtchromatographie gereinigt werden.

**Patentansprüche**

1. Tridekapeptid der Formel

H-Met-Ser-Tyr-Asn-Leu-Leu-Gly-Phe-Leu-Gln-Arg-Ser-Ser-OH

2. $Arg^{11}$-(Tos)-Tridekapeptid der Formel

H-Met-Ser-Tyr-Asn-Leu-Leu-Gly-Phe-Leu-Gln-Arg(Tos)-Ser-Ser-OH

3. Tridekapeptid gemäß Anspruch 1 zur Verwendung als Hapten, Tracer oder Antikörper.

4. Verfahren zur Herstellung des Tridekapeptids der Formel

H-Met-Ser-Tyr-Asn-Leu-Leu-Gly-Phe-Leu-Gln-Arg-Ser-Ser-OH

dadurch gekennzeichnet, daß man in einem Lösungsmittel die C-terminale geschützte Aminosäure an ein Polymer als unlöslichen Träger anknüpft, nach jeweiliger Abspaltung das Peptid durch stufenweisen Anbau der einzelnen gegebenenfalls in der Seitenfunktion geschützten N-geschützten Aminosäuren an das jeweilige Aminoende des Peptidfragments am polymeren Träger gemäß obiger Formel aufbaut, wobei man vir jedem neuen Synthese-Zyklus gegebenenfalls noch vorhandene freie Aminogruppen durch Acetylierung blockiert und anschließend die verwendete Aminoschutzgruppe abspaltet, und anschließend von dem durch Behandeln des so erhaltenen voll geschützten Peptid-Harzes mit Bromwasserstoff erhaltenen $N^g$-Tosyl-tridekapeptid die Tosylgruppe durch Behandeln mit Alkalimetall/Ammoniak abspaltet und das so erhaltene Tridekapeptid nach an sich bekannten Methoden reinigt.

5. Verfahren gemäß Anspruch 4, dadurch gekennzeichnet, daß man als Aminoschutzgruppe die tert.Butyloxycarbonylgruppe verwendet.

6. Verfahren gemäß Anspruch 4, dadurch gekennzeichnet, daß man die Seitenfunktionen der Aminosäuren Serin, Tyrosin und Arginin durch Überführung in entsprechende O-Benzylserin-O-2,6-Dichlorbenzyltyrosin- und $N^g$-Tosyl-arginin-Derivate schützt.

7. Verfahren gemäß Anspruch 4, dadurch gekennzeichnet, daß man Serin als C-terminale Aminosäure in seiner geschützten Form über ihr Cäsium-Salz an chlormethyliertes Polystyrol anknüpft.

8. Verfahren gemäß Anspruch 4, dadurch gekennzeichnet, daß man die 2., 3., 5. bis 9. und 11. bis 13. N-geschützte Aminosäure durch Aktivierung der Carboxylfunktion mittels eines Kupplungsreagenzes wie N,N'-Dicyclohexylcarbodiimid anknüpft.

9. Verfahren gemäß Anspruch 4, dadurch gekennzeichnet, daß man die 4. und 10. N-geschützte Aminosäure über einen aktivierten Ester, z. B. über ihren p-Nitrophenylester, anknüpft.

10. Verfahren gemäß den Ansprüchen 4 bis 9, dadurch gekennzeichnet, daß man jeden Synthese-Zyklus mit einem Überschuß, z. B. mit der 3- bis 6-fachen Menge, der jeweiligen geschützten Aminosäure durchführt und dies 2 bis 5 mal wiederholt.

11. Verfahren gemäß den Ansprüchen 4 bis 9, dadurch gekennzeichnet, daß man den Boc-Schutzrest durch Behandeln mit 50 %iger Trifluoressigsäure in Dichlormethan abspaltet.

12. Verfahren gemäß den Ansprüchen 4 bis 11, dadurch gekennzeichnet, daß die Anknüpfung der

geschützten Aminosäuren in Gegenwart eines Katalysators, z. B. in Gegenwart von 1-Hydroxybenzotriazol, durchgeführt wird.

## Claims

1. Tridecapeptide of formula

H-Met-Ser-Tyr-Asn-Leu-Leu-Gly-Phe-Leu-Gln-Arg-Ser-Ser-OH

2. Arg[11]-(Tos)-tridecapeptide of formula

H-Met-Ser-Tyr-Asn-Leu-Leu-Gly-Phe-Leu-Gln-Arg(Tos)-Ser-Ser-OH

3. Tridecapeptide as claimed in claim 1 for use as a hapten, tracer or antibody.
4. Process for preparing the tridecapeptide of formula

H-Met-Ser-Tyr-Asn-Leu-Leu-Gly-Phe-Leu-Gln-Arg-Ser-Ser-OH

characterised in that the C-terminal protected amino acid is linked to a polymer as an insoluble carrier in a solvent, after the amino protecting group has been splitt off the peptide is synthesised on the polymeric carrier according to the above formula by stepwise addition of the individual N-protected amino acids, optionally protected in the side function, to the amino end the peptide fragment, and before each new cycle of synthesis any free amino groups which may still be present are blocked by acetylation and subsequently the amino protecting group used is split off, and subsequently the tosyl group is split off, by treatment with alkali metal/ammonia, from the $N^g$-tosyl-tridecapeptide obtained by treating the resultant fully protected peptide resin with hydrogen bromide, and the tridecapeptide thus obtained is purified by methods known *per se.*

5. Process as claimed in claim 4, characterised in that the tert.butyloxycarbonyl group is used as the amino protecting group.
6. Process as claimed in claim 4, characterised in that the side functions of the amino acids serine, tyrosine and arginine are protected by converting them into corresponding O-benzylserine, O-2,6-dichlorobenzyltyrosine and $N^g$-tosyl-arginine derivatives.
7. Process as claimed in claim 4, characterised in that serine, as a C-terminal amino acid, is linked to chloromethylated polystyrene in its protected form via its caesium salt.
8. Process as claimed in claim 4, characterised in that the 2nd, 3rd, 5th to 9th and 11th to 13th N-protected amino acids are linked by activating the carboxyl function using a coupling reagent such as N,N'-dicyclohexylcarbodiimide.
9. Process as claimed in claim 4, characterised in that the 4th and 10th N-protected amino acids are linked via an activated ester, e. g. their p-nitrophenyl esters.
10. Process as claimed in claims 4 to 9, characterised in that each cycle of synthesis is carried out with an excess, e. g. with 3 to 6 times the quantity, of the protected amino acid in question and this is repeated 2 to 5 times.
11. Process as claimed in claims 4 to 9, characterised in that the Boc protecting group is split off by treatment with 50 % trifluoroacetic acid in dichloromethane.
12. Process as claimed in claims 4 to 11, characterised in that the linking of the protected amino acids is carried out in the presence of a catalyst, e. g. in the presence of 1-hydroxy-benzotriazole.

## Revendications

1. Tridécapeptide de formule

H-Met-Ser-Tyr-Asn-Leu-Leu-Gly-Phe-Leu-Gln-Arg-Ser-Ser-OH

2. Arg[11]-(Tos)-tridécapeptide de formule

H-Met-Ser-Tyr-Asn-Leu-Leu-Gly-Phe-Leu-Gln-Arg(Tos)-Ser-Ser-OH

3. Tridécapeptide selon la revendication 1, pour l'utilisation en tant que haptène, traceur ou anticorps.
4. Procédé pour la préparation du tridécapeptide de formule

H-Met-Ser-Tyr-Asn-Leu-Leu-Gly-Phe-Leu-Gln-Arg-Ser-Ser-OH

caractérisé en ce que dans un solvant on relie l'acide aminé protégé C-terminal à un polymère en tant que support insoluble, après clivage respectif du radical protecteur de l'amino on construit le peptide selon la formule ci-dessus par adjonction par étapes des amino-acides particuliers, N-protégés, éventuellement protégés dans la fonction latérale, sur l'extrémité amino respective du fragment peptidique sur le support polymère, en bloquant par acétylation, avant chaque nouveau cycle de synthèse, les groupes amino libres éventuellement encore présents et en clivant ensuite les groupes protecteurs de l'amino utilisés, et en ce qu'on clive ensuite par traitement avec un métal alcalin/ammoniac le groupe tosyle à partir du $N^g$-tosyl-tridécapeptide obtenu par traitement de la résine munie du peptide complètement protégée ainsi obtenue avec du bromure d'hydrogène et en ce qu'on purifie le tridécapeptide ainsi obtenu selon les méthodes connues en soi.

5. Procédé selon la revendication 4, caractérisé en ce que l'on utilise en tant que groupe protecteur de l'amino le groupe tert-butyloxycarbonyle.

6. Procédé selon la revendication 4, caractérisé en ce qu'on protège les fonctions latérales des amino-acides sérine, tyrosine et arginine par transformation en les dérivés de O-benzylsérine, O-2,6-dichlorobenzyltyrosine et $N^g$-tosyl-arginine correspondants.

7. Procédé selon la revendication 4, caractérisé en ce qu'on fixe la sérine en tant qu'amino-acide C-terminal sous sa forme protégée par l'intermédiaire de son sel de césium sur du polystyrène chlorométhylé.

8. Procédé selon la revendication 4, caractérisé en ce qu'on fixe les 2$^e$, 3$^e$; 5$^e$ à 9$^e$ et 11$^e$ à 13$^e$ amino-acides protégés sur N par activation de la fonction carboxyle au moyen d'un réactif de couplage tel que le N,N'-dicyclohexylcarbodiimide.

9. Procédé selon la revendication 4, caractérisé en ce qu'on fixe les 4$^e$ et 10$^e$ amino-acides protégés sur N par l'intermédiaire d'un ester activé, par exemple par l'intermédiaire de leurs esters p-nitrophényliques.

10. Procédé selon les revendications 4 à 9, caractérisé en ce qu'on effectue chaque cycle de synthèse au moyen d'un excès, par exemple avec une quantité de 3 à 6 fois, de l'amino-acide protégé respectif et répète ceci 2 à 5 fois.

11. Procédé selon les revendications 4 à 9, caractérisé en ce qu'on clive le radical protectecteur Boc par traitement avec de l'acide trifluoroacétique à 50 % dans le dichlorométhane.

12. Procédé selon les revendications 4 à 11, caractérisé en ce que la fixation des amino-acides protégés est effectuée en présence d'un catalyseur, par exemple en présence de 1-hydroxy-benzotriazole.

Boc-Ser(Bzl)-O$^{\ominus}$Cs$^{\oplus}$ Cl-$CH_2$-PS-1%DVB

48h in DMF

Boc-Ser(Bzl) -$OCH_2$-PS

1. $CF_3COOH/CH_2Cl_2$

2. $N(C_2H_5)_3/CHCl_3$

Boc-Ser(Bzl)-OH+H-Ser(Bzl) -$OCH_2$-PS

DCC/HOBt in $CH_2Cl_2$

Boc-Ser(Bzl)-Ser(Bzl)-$OCH_2$-PS

Boc-Gln-ONp+H-Arg(Tos)-Ser(Bzl)-Ser(Bzl)-$OCH_2$-PS

HOBt, DMF

Boc-Gln-Arg(Tos)-Ser(Bzl)-Ser(Bzl)-$OCH_2$-PS

( 5x )

Boc-Leu-Leu-Gly-Phe-Leu-Gln-Arg(Tos)-Ser(Bzl)-Ser(Bzl)-$OCH_2$-PS

Boc-Asn-ONp+H-Leu-Leu-Gly-Phe-Leu-Gln-Arg(Tos)-Ser(Bzl)-Ser(Bzl)-$OCH_2$-PS

HOBt, DMF

Boc-Asn -Leu-Leu-Gly-Phe-Leu-Gln-Arg(Tos)-Ser(Bzl)-Ser(Bzl)-$OCH_2$-PS

( 3x )

Boc-Met-Ser(Bzl)-Tyr(2,6-$Cl_2$-Bzl)-Asn -Leu-Leu-Gly-Phe-Leu-Gln-Arg(Tos)-Ser(Bzl)-Ser(Bzl)-$OCH_2$-PS

$HBr/CF_3COOH$, 90 min

H-Met-Ser-Tyr-Asn -Leu-Leu-Gly-Phe-Leu-Gln-Arg(Tos)-Ser-Ser-OH

Na, $NH_3$ fl.

H-Met-Ser-Tyr-Asn--Leu-Leu-Gly-Phe-Leu-Gln-Arg- - Ser-Ser-OH

Abb. 1

Abb. 2

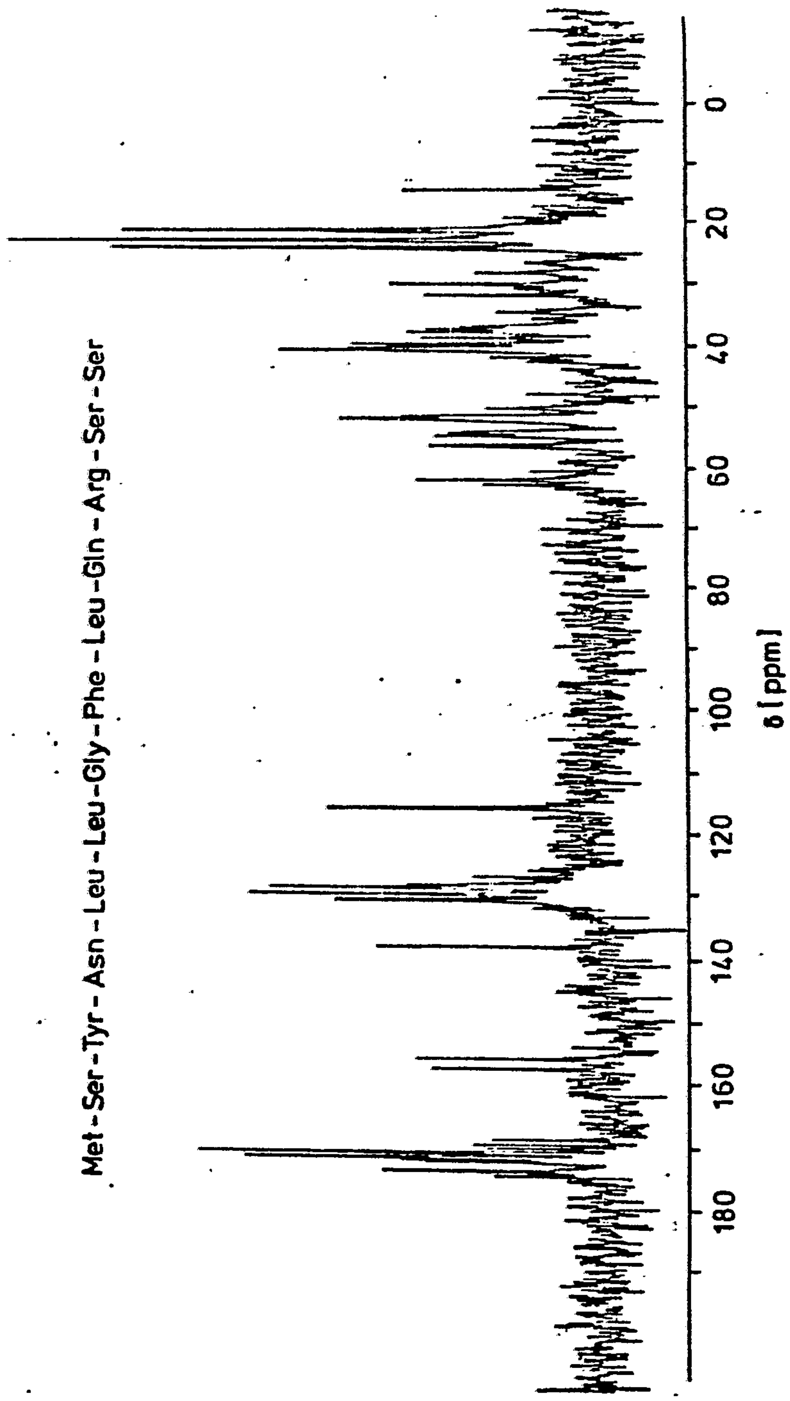

Met-Ser-Tyr-Asn-Leu-Leu-Gly-Phe-Leu-Gln-Arg-Ser-Ser
0
20
40
60
80
100
120
140
160
180
δ [ppm]

Abb.3a

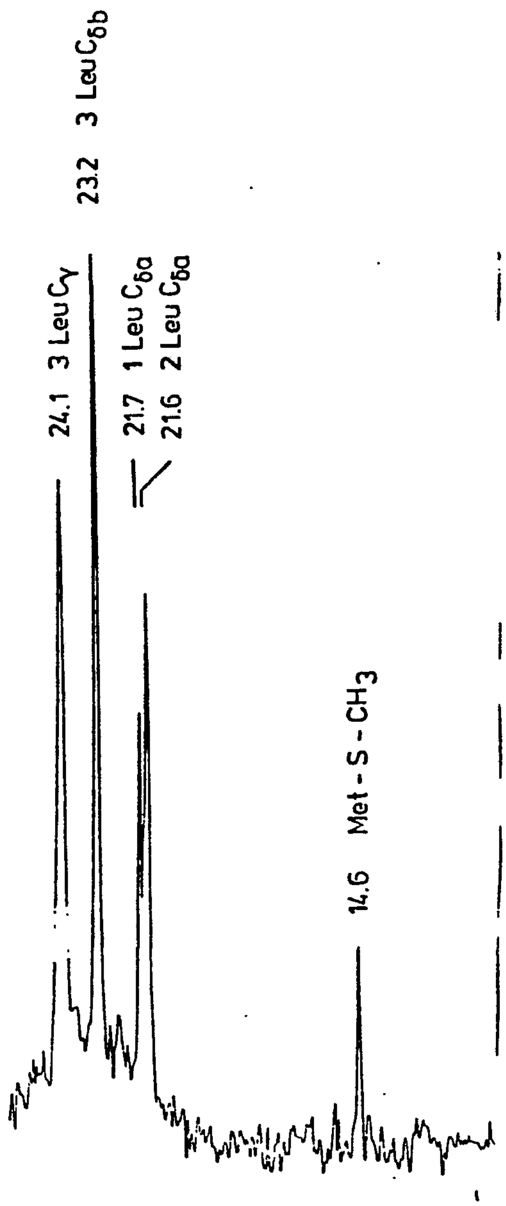

Abb. 3b

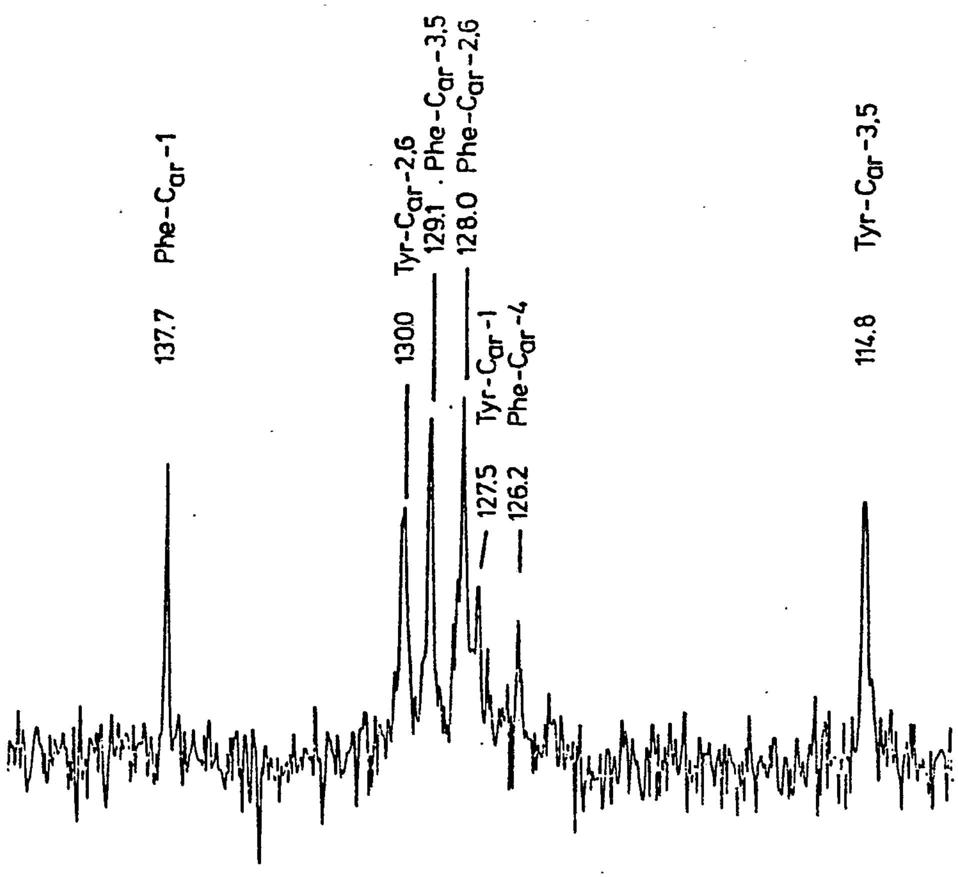
137.7 Phe-$C_{ar}$-1
130.0 Tyr-$C_{ar}$-2,6
129.1 . Phe-$C_{ar}$-3,5
128.0 Phe-$C_{ar}$-2,6
127.5 Tyr-$C_{ar}$-1
126.2 Phe-$C_{ar}$-4
114.8 Tyr-$C_{ar}$-3,5

Abb. 3c

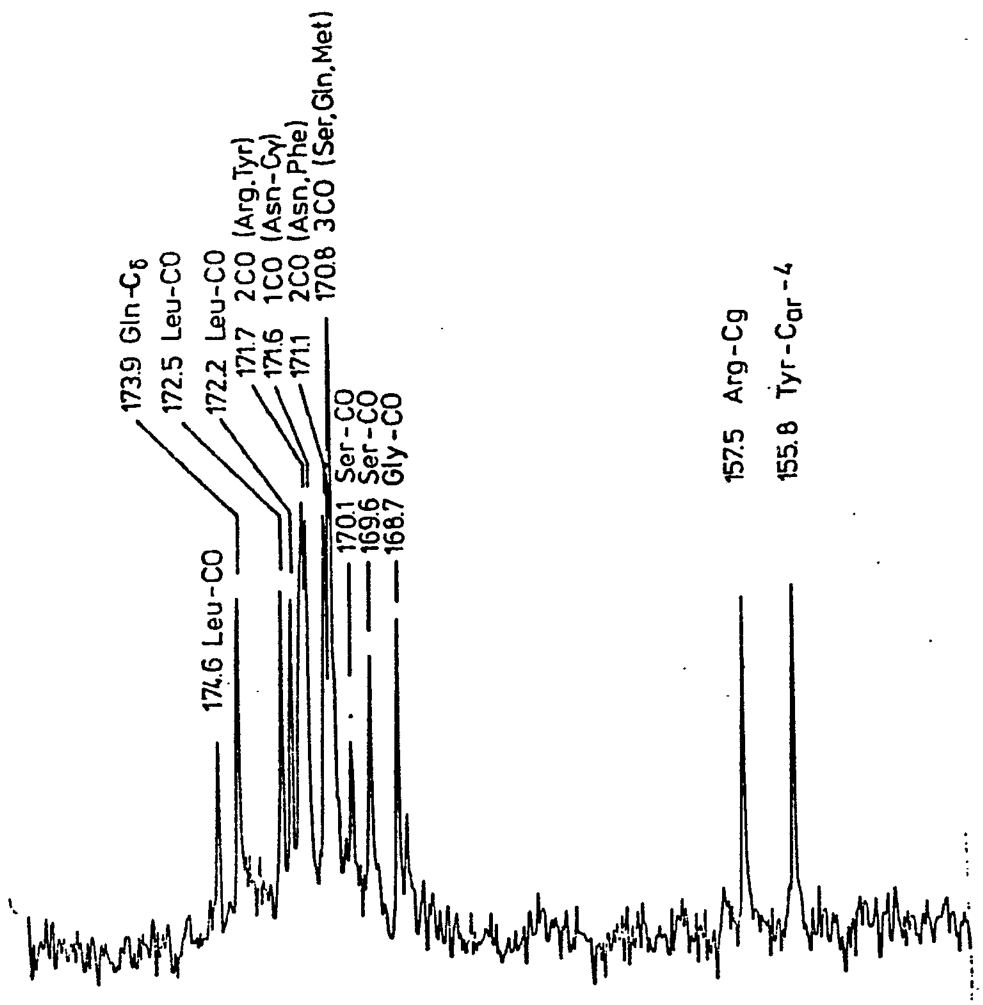
174.6 Leu-CO
173.9 Gln-$C_\delta$
172.5 Leu-CO
172.2 Leu-CO
171.7 2CO (Arg,Tyr)
171.6 1CO (Asn-$C_\gamma$)
171.1 2CO (Asn,Phe)
170.8 3CO (Ser,Gln,Met)
170.1 Ser-CO
169.6 Ser-CO
168.7 Gly-CO
157.5 Arg-$C_\xi$
155.8 Tyr-$C_{ar}$-4

Abb.4: Gaschromatogramm der N-Pentafluorpropionylaminosäure-n-propylester eines Hydrolysats des freien Tridekapeptids an chiraler Phase.